(19) Europäisches Patentamt
European Patent Office
Office européen des brevets

(11) Numéro de publication: 0 424 242 A2

(12) **DEMANDE DE BREVET EUROPEEN**

(21) Numéro de dépôt: 90402890.9

(22) Date de dépôt: 16.10.90

(51) Int. Cl.5: C07C 51/285, C07C 51/29

(30) Priorité: 16.10.89 FR 8913472

(43) Date de publication de la demande: 24.04.91 Bulletin 91/17

(84) Etats contractants désignés: AT BE CH DE ES FR GB IT LI LU NL SE

(71) Demandeur: L'AIR LIQUIDE, SOCIETE ANONYME POUR L'ETUDE ET L'EXPLOITATION DES PROCEDES GEORGES CLAUDE
75, Quai d'Orsay
F-75321 Paris Cédex 07(FR)

(72) Inventeur: Longeray, Rémi
2 chemin de Montbel
F-69390 Vourles(FR)
Inventeur: Lanteri, Pierre
25B rue Georges Courteline
F-69100 Villeurbanne(FR)
Inventeur: Lu, Xiao
3 rue des Onchères, Appartement 1246
F-69120 Vaulx en Velin(FR)
Inventeur: Huet, Catherine
50 route de Givry, Saint Rémy
F-71100 Chalons sur Saône(FR)

(74) Mandataire: Le Moenner, Gabriel et al
L'AIR LIQUIDE, Société Anonyme pour l'étude et l'exploitation des procédés Georges Claude 75, Quai d'Orsay
F-75321 Paris Cédex 07(FR)

(54) **Procédé de fabrication d'acides carboxyliques par oxidation des aldehydes correspondants.**

(57) Le procédé de fabrication d'acides carboxyliques de l'invention comprend l'oxydation d'aldéhydes à l'aide d'un composé peroxydé, en phase liquide homogène, en présence de quantités catalytiques d'acide halohydrique.

EP 0 424 242 A2

# PROCEDE DE FABRICATION D'ACIDES CARBOXYLIQUES PAR OXYDATION DES ALDEHYDES CORRESPONDANTS

La présente invention a pour objet un procédé de fabrication d'acides carboxyliques par oxydation des aldehydes correspondants.

De nombreux oxydants ont été proposés pour préparer industriellement les acides à partir des aldéhydes. Les réactifs les plus utilisés sont l'oxygène, le permanganate, l'oxyde d'argent, l'acide chromique, l'eau oxygénée et les peracides.

Les oxydations d'aldéhydes par les oxydants inorganiques tels que l'oxygène, l'acide chromique, le permanganate, l'oxyde d'argent, le tétra-acétate de plomb et le dioxyde de manganèse présentent plusieurs inconvénients :

- nécessité d'employer des quantités stoechiométriques et souvent de larges excès d'oxydants par rapport à l'aldéhyde,
- problèmes de toxicité impliquant l'élimination totale de traces métalliques à cause de l'utilisation finale visée et conditions drastiques liées à l'environnement;

ces deux points ayant un impact important sur l'économie du procédé,

- manque de sélectivité abaissant le rendement en acide carboxylique : de nombreux sous-produits sont obtenus dans les conditions fortement acides ou basiques employées qui provoquent une coupure oxydante des liaisons C-C des aldéhydes énolysables.

Pour s'affranchir de ces problèmes, l'emploi de dérivés peroxydés a été proposé.

Les oxydations par $H_2O_2$ en présence de catalyseurs métalliques du type $CrO_3$, $V_2O_5$, $SnO_2$, $CeO_2$, $FeCl_3$, $H_2WO_4$ et $H_2MoO_4$ n'ont souvent pas lieu ou conduisent à de nombreux sous-produits.

Le document GB - A- 2 004 543 revendique l'utilisation de catalyseurs à base de sélénium avec $H_2O_2$ ou l'hydroperoxyde de tertiobutyle permettant d'obtenir de très hauts rendements en acide. La toxicité élevée de ce métal et l'utilisation finale du produit formé rendent nécessaire d'éliminer totalement toutes traces de sélénium.

Le peroxyde d'hydrogène catalysé soit par les acides soit par les bases réagit sur les aldéhydes avec formation d'$\alpha$-hydroxyhydroperoxydes intermédiaires qui conduisent à de nombreux sous-produits. Il s'ensuit que le rendement en acide carboxylique correspondant est souvent faible.

Les peroxyacides (acide de Caro, acide performique, acide peracétique) oxydent les aldéhydes aliphatiques linéaires et le benzaldéhyde en donnant des rendements élevés en acides carboxyliques correspondants.

Sur des aldéhydes suffisamment riches en électrons favorisant la migration du groupement en $\alpha$ du carbonyle et notamment sur les aldéhydes aromatiques avec des groupements électrodonneurs (OH, $OCH_3$, ...) en ortho et para sur le noyau, le réarrangement de BAYER VILLIGER est en compétition avec la formation de l'acide carboxylique. Le clivage de la liaison C-C puis la migration du groupement $R_1$ sur l'atome d'oxygène du peracide conduit au formiate:

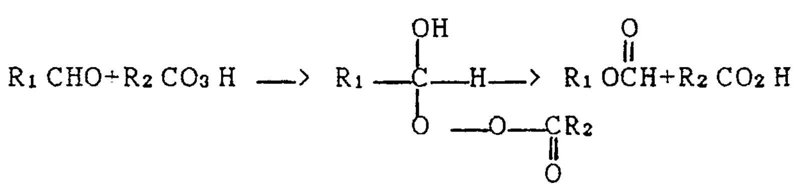

L'oxydation des alcools secondaires en cétones par le chlore ou le brome a déjà été largement étudiée (réf. PERLMUTTER - HAYMAN B. et al, J. of Amer. Chem. Soc. 91:3, 1969, p.668 et GARDNER - SWAIN, J. of Amer. Soc. 83, 1961, p.1945).

Le brevet européen 260054 d'INTEROX de 1987 décrit l'oxydation d'alcools en cétones par utilisation du réactif $H_2O_2$-HBr.

Mais pour améliorer la sélectivité, il est préconisé d'utiliser un système biphasique et pour générer des radicaux Br une irradiation des réactifs par des lampes dont les émissions principales sont dans la gamme de longueurs d'onde de 250 à 600 nm.

De plus, le procédé décrit n'est pas catalytique puisqu'il ne fonctionne que pour des rapports molaires $Br^-$ : alcools sélectionnés dans le domaine 0,4 : 1 à 1,5 : 1.

Ce procédé est recommandé pour l'oxydation sélective d'alcools substitués par des groupements électronégatifs adjacents du type halopropanols.

La demande de brevet EP 249289A publiée le 16.12.87 revendique l'oxydation de phénols en quinones par $H_2O_2$ catalysé par HBr ou HI.

La demande de brevet EP 278107A publiée le 17.8.88 revendique l'oxydation de la fonction aldéhydique des aldoses en acides aldoniques par $H_2O_2$ catalysé par l'iode ou un composé contenant de l'iode.

L'objet de la présente invention est de fournir un procédé de préparation d'acides carboxyliques permettant de remédier aux inconvénients des procédés de l'art antérieur, de mise en oeuvre aisée, utilisable à l'échelle industrielle.

L'invention vise en particulier à fournir un procédé d'oxydation particulièrement adapté aux aldéhydes susceptibles de subir des réactions de BAEYER VILLIGER.

Le procédé de préparation d'acides carboxyliques par oxydation des aldéhydes correspondants est caractérisé en ce qu'on opère en phase liquide homogène, en présence de quantités catalytiques d'acide halohydrique, en utilisant comme agent oxydant un composé peroxydé capable d'oxyder l'acide halohydrique en libérant l'halogène, et qu'on récupère l'acide carboxylique formé.

Sans pour autant lier l'invention à un quelconque mécanisme, les inventeurs considèrent que la première étape consiste en une réaction du composé peroxydé sur l'acide halohydrique avec libération de l'halogène, suivie de l'oxydation de l'aldéhyde par l'halogène libéré, avec régénération de l'acide halohydrique à nouveau réoxydé, qui peut donc être utilisé en quantité catalytique.

Tout se passe comme si ce système se comportait comme une oxydation classique par un halogène tout en présentant les avantages suivants :

- ne pas produire d'effluents acides,
- éviter l'utilisation directe de l'halogène dont la manipulation à l'échelle industrielle est malcommode, et dangereuse et crée des problèmes pour l'environnement. On sait par exemple que le brome est un liquide toxique de bas point d'ébullition, qui réagit avec l'eau pour former des vapeurs toxiques et corrosives,
- ne pas s'accompagner d'une forte consommation d'halogène,
- permettre d'obtenir de très bonnes sélectivités sans réactions parasites de substitution par l'halogène.

L'équation réactionnelle globale du procédé selon l'invention peut s'écrire :

$RCHO + H_2O_2 \rightarrow R\text{-}COOH + H_2O$

Les aldéhydes susceptibles d'être soumis à la réaction d'oxydation conforme à la présente invention présentent la formule suivante :

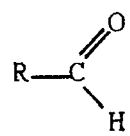

dans laquelle R représente

- un groupe hydrocarboné de 1 à 30 atomes de carbone, linéaire ou ramifié, comportant le cas échéant dans la chaîne, un ou plusieurs atomes d'oxygène et/ou un ou plusieurs groupes oxo, choisi parmi les groupes alcoyle, alcoxy ou acyle,
- un groupe cycloalcoyle de 1 à 30 atomes de carbone, un groupe aryle comprenant un ou plusieurs cycles aromatiques,
- un groupe arylacyle où les parties aryle et acyle sont respectivement telles que définies ci-dessus,
- un groupe hétérocyclique;

ces différents groupes étant le cas échéant substitués par un ou plusieurs groupements inertes dans les conditions réactionnelles, tels que des atomes d'halogène, des groupes alcoyle, alcoxy dans lesquels la partie alcoyle, linéaire ou ramifiée, comporte de 1 à 5 atomes de carbone, -COOA dans lequel A représente un groupe alcoyle de 1 à 5 atomes de carbone, aryloxy, benzo et nitro.

Les groupes alcoyle comprennent notamment de 1 à 5 atomes de carbone. Les groupes aryle sont notamment choisis parmi les groupes phényle et naphtyle. Comme hétérocycle, on citera entre autres le benzoxazole.

Ces aldéhydes sont des produits commerciaux ou sont synthétisables selon les réactions classiques de la chimie organique,par exemple en ayant recours à l'oxydation d'hydrocarbures saturés ou insaturés, de dérivés monohalogénés d'hydrocarbures saturés ou aromatiques, d'alcools primaires. Il est également connu d'hydrolyser des dérivés gem-dihalogénés primaires, des aldimines, de déshydrater des $\alpha$-glycols ou d'oxyder des $\alpha$-glycols bisencondaires.

D'autres techniques largement utilisées comprennent la formylation selon les procédés oxo et GATTERMAN-KOCH, la réduction du type ROSENMUND, la décomposition des acides α-alcools, ou encore la mise en oeuvre de la méthode de DARZENS.

Des aldéhydes convenant tout particulièrement à l'oxydation conforme à la présente invention sont ceux qui, soumis à l'action l'un peroxyacide préformé ou généré in situ dans le milieu réactionnel, subissent le réarrangement de BAEYER-VILLIGER.

On peut citer à titre d'exemple non limitatif :

- des aryl-2-propanal différemment substitués, par exemple:
phényl-2-propanal,
(alkyl-4-phényl)2-propanal, tels que (méthyl-4-phényl)2-propanal, (isobutyl-4-phényl)2-propanal,
(alkényl-4-phényl)2-propanal,
(méthoxy-4-phényl)2-propanal,
(chloro-4 ou bromo-4-phényl)2-propanal,
(nitro-4-phényl)2-propanal,
naphtyl-2-propanal,
(méthoxy-6-naphtyl)-2-propanal,
(phénoxy-3-phényl)2-propanal,
(benzo-3-phényl)2-propanal,
(fluoro-3-diphényl)2-propanal,
(chloro-4-phénylbenzoxazolyl)2-propanal,
(benzoyl-3-phényl)2-propanal,
- aryl éthanal différemment substitués (par exemple : phényl éthanal et les dérivés substitués correspondants, diphényl éthanal, triphényl éthanal).

D'autres aldéhydes particulièrement intéressants pour la présente invention sont les :

- benzaldéhydes mono et pluri substitués tels que les alcoyl benzaldéhydes, (par exemple isopropyl-4-benzaldéhyde), les dichlorobenzaldéhydes (par exemple dichloro-2,6-benzaldéhyde), les trifluoro benzaldéhydes (par exemple trifluoro-ααα-para, méta et ortho tolualdéhyde),
- aldéhydes aliphatiques tels que éthanal, glyoxal, propanal, n-butanal, isobutanal, n et iso pentanal, hexanal, heptanal, octanal,
- aldéhydes obtenus par hydroformylation des oléfines C10-C30 en particulier l'aldéhyde oxo en $C_{15}$.

Le composé peroxydé est choisi parmi les composés capables d'oxyder les acides halohydriques en halogène correspondant et notamment parmi :

- le peroxyde d'hydrogène,
- un peroxyde capable de libérer du peroxyde d'hydrogène en milieu aqueux, tel l'anion peroxyborate,
- les hydroperoxydates et, en particulier, les dérivés de l'eau oxygénée avec l'urée ou des sels alcalins et alcalino-terreux des acides par exemple carboniques, siliciques ou pyrophosphoriques.
- les sels de l'acide diperoxysulfurique,
- les peroxyacides organiques, l'acide monoperoxysulfurique et ses sels (dans les cas où le réarrangement de BAEYER VILLIGER n'est pas la réaction majoritaire),
- les composés à fonction hydroperoxyde tels que par exemple l'hydroperoxyde de tertiobutyle.

Ces composés peroxydés sont des produits disponibles dans le commerce ou peuvent être obtenus aisément selon les méthodes habituelles de synthèse organique.

Les différents paramètres intervenant dans la réaction d'oxydation de l'invention en particulier les concentrations relatives en réactifs, le pH, la température seront aisément mis au point par l'homme de l'art, en ayant recours aux techniques classiques, compte tenu des produits utilisés et des problèmes de solubilité rencontrés. De façon générale, on met en oeuvre le composé peroxydé selon des quantités au moins steochiométriques par rapport à l'aldéhyde.

Dans le cas par exemple de l'utilisation du peroxyde d'hydrogène, les concentrations recommandées varient de 1 à 85% en poids et, préférentiellement, de 30 à 35% environ.

Le rapport molaire $H_2O_2$/aldéhyde peut varier dans le domaine 0,5/1 à 3/1 environ, il est préférentiellement de l'ordre de 2/1.

L'acide halohydrique HX est choisi avantageusement parmi HCl ou HBr. On l'utilise soit sous forme concentrée, soit à l'état dilué. Il est cependant préférable de l'utiliser concentré : à 47%, en poids, environ pour HBr et à 37% environ, en poids, pour HCl.

Le rapport molaire HX : aldéhyde sera au minimum de 0,005 : 1 et préférentiellement de l'ordre de 0,09 : 1 0,39 : 1.

De manière générale, la réaction d'oxydation est réalisée à pH acide.

Dans le cas d'utilisation d'un peroxyde minéral ou d'un hydroperoxydate à réaction alcaline en solution

aqueuse, le pH est diminué jusqu'à une valeur inférieure ou égale à 5.

Les solvants organiques employés doivent être tels que le milieu réactionnel soit homogène. De plus, ils doivent être compatibles et inertes vis-à-vis des réactifs utilisés.

Les meilleurs rendements sont obtenus dans les solvants à fonction éther tels que le dioxanne 1-4, le tétrahydrofuranne, l'éther éthylique mais à titre d'exemples non limitatifs, on peut encore citer les alcools aliphatiques tertiaires (par exemple l'alcool tert butylique, tert amylique), les glymes (par exemple: les éthylène glycols mono ou diméthyl éther) l'acide acétique, l'acétonitrile ou le nitrométhane.

Les valeurs convenables de la concentration de l'aldéhyde dans le solvant organique sont comprises entre 5% et environ 95% en poids, et sont préférentiellement de l'ordre de 25%.

Les temps de réaction nécessaires pour que la conversion en aldéhyde soit totale varient largement en fonction de la température de réaction mais ils sont habituellement compris entre 15 mn et 48 h, et le plus souvent de l'ordre de 2 à 5 h.

On a constaté que des températures de réaction comprises entre 5 et 100° C et de préférence vers 40-60° C, et des pressions comprises entre la pression atmosphérique et quelques bars, notamment de l'ordre de 6 bars sont en général satisfaisantes.

L'acide carboxylique obtenu est séparé selon les méthodes classiques à la fin du temps de réaction requis ; le solvant organique est évaporé et récupéré pour recyclage ; le résidu est repris dans un solvant non miscible à l'eau. La phase organique est alors lavée, avantageusement à plusieurs reprises avec une solution réductrice, par exemple sulfite, thiosulfate pour éliminer les peroxydes restants.

L'acide est soit précipité puis recristallisé, soit distillé sous pression réduite.

La mise en oeuvre des dispositions qui précèdent conduit à l'obtention de rendements en acides carboxyliques élevés, pouvant atteindre 80% ou plus.

## EXEMPLES D'APPLICATION

Les exemples suivants sont décrits pour illustrer l'invention sans en limiter la portée.

L'exemple 7 est donné à titre de comparaison.

### EXEMPLE 1

Dans un ballon muni d'un agitateur magnétique sont introduits: 50 ml de dioxanne, 13,4g (0,1M) de phényl-2-propanal (produit commercial), 1 ml d'HBr 47% (0,009M) et 10 ml d'$H_2O_2$ à 35% (0,12M). Le mélange réactionnel est porté à 40° C et est maintenu à cette température pendant 5 h.

L'analyse par chromatographie liquide révèle la formation de 82% d'acide phényl-2-propanoïque.

La conversion de l'aldéhyde est de 98%. Le sous-produit majoritaire obtenu est l'acétophénone.

Le dioxanne et évaporé, le résidu est repris dans l'éther, lavé avec une solution à 5% de sulfite de sodium. La phase organique est séchée sur sulfate. Le solvant est évaporé.

L'acide phényl-2-propanoïque est distillé sous pression réduite à 0,6 mbar à 110° C.

### EXEMPLES 2 à 6

Le protocole utilisé est identique à celui décrit dans l'exemple 1, la température variant de 20 à 65° C et la nature du solvant pouvant différer.

On rapporte dans le tableau qui suit les rendements en acide obtenus en fonction des conditions réactionnelles.

| EXEMPLES | SOLVANT | TEMPERATURE | TEMPS REACTION | RENDEMENT ACIDE |
|---|---|---|---|---|
| 2 | Dioxanne | 50° C | 2 h | 79% |
| 3 | tBuOH | 40° C | 6 h | 50% |
| 4 | Ethylène glycol diméthyléther | 40° C | 6 h | 53% |
| 5 | Nitrométhane | 40° C | 5 h | 50% |
| 6 | Tert amylalcool | 40° C | 6 h | 57% |

***EXEMPLE 7***

L'exemple 3 a été repris sans introduction de HBr. L'acide phényl-2-propanoïque n'est pas détecté après plus de 6 heures de réaction.

***EXEMPLE 8***

Dans un ballon muni d'un agitateur magnétique sont introduits : 50 ml d'alcool tert amylique, 13,4g (0,1M) de phényl-2-propanal, 2,5 ml de HBr 74% (0,0225M) et 17,5ml d'$H_2O_2$ à 35% (0,2M). Le mélange réactionnel est porté à 40° C pendant 6h.

L'acide phényl-2-propanoïque est obtenu en fin de réaction avec un rendement de 77% (analyse CPG).

***EXEMPLE 9***

Dans un réacteur double enveloppe muni d'un agitateur mécanique et d'un serpentin réfrigérant interne, sont introduits 50 ml de dioxanne, 13,4g de phényl-2-propanal (0,1M), 1,5ml d'HBr 47% (0,0135M) et 15 ml d'$H_2O_2$ 35% (0,18M). Le mélange réactionnel est porté à 40° C pendant 6 h.

L'acide phényl-2-propanoïque est obtenu en fin de réaction avec un rendement de 89% (analyse CPG).

***EXEMPLES 10 à 15***

On opère comme décrit précédemment en utilisant les aldéhydes de départ mentionnés dans le tableau qui suit, dans lequel on indique les rendements en acide.

| Exemple | Aldéhyde | Conditions | Rendement acide |
|---|---|---|---|
| 10 | (isobutyl-4-phényl)2 propanal (préparé selon le brevet GB 1160725) | 40° C - 6 h 1,14 g aldéhyde 0,009 ml HBr 0,9 ml $H_2O_2$ Dioxanne 3ml | 70 % |
| 11 | n butanal | Dioxanne | 90 % |
| 12 | n octanal | Dioxanne | 96 % |
| 13 | isopropyl-4-benzaldéhyde | Dioxanne 10 mM aldéhyde 4 h 60° C 0,15 ml HBr 1,5 ml $H_2O_2$ | 50 % |
| 14 | dichloro-2,6-benzaldéhyde | Dioxanne 10 mM aldéhyde 4 h 60° C 0,15 ml HBr 1,5 ml $H_2O_2$ | 54 % |
| 15 | trifluoro-$\alpha\alpha\alpha$-tolualdéhyde | 4 h 60° C Dioxanne | 55 % |

L'acide obtenu selon l'exemple 10, connu sous le nom générique ibuprofen présente l'avantage de présenter des propriétés anti-inflammatoires le rendant utile en thérapeutique.

Les résultats rapportés ci-dessus mettent en évidence ls avantages du procédé d'oxydation de l'invention.

Ce procédé présente un intérêt tout particulier pour l'obtention d'acides carboxyliques de valeur dans différents domaines industriels. A titre d'exemple, on citera le domaine pharmaceutique dans lequel sont utilisés de nombreux acides carboxyliques en tant que principes actifs de médicaments. Comme illustré par l'exemple 10 ci-dessus, les acides arylacétiques et arylproprioniques présentent aussi des propriétés antiinflamatoires et sont largement utilisés en tant qu'agents antiphlogistiques. Des acides de ce type sont connus sous les noms génériques suivants : flurbiprofen, fenoprofen, ketoprofen, naproxen, benoxaprofen

ou encore fenbufen.

Il est clair que les acides seront élaborés selon les applications recherchées, en utilisant l'aldéhyde possédant le groupement R approprié.

## Revendications

1. Procédé de préparation d'acides carboxyliques par oxydation des aldéhydes correspondants, caractérisé en ce qu'on utilise des aldéhydes alpha-substitués et qu'on opère en phase liquide homogène, en présence de quantités catalytiques d'acide halohydrique, en utilisant comme agent oxydant un composé peroxydé capable d'oxyder l'acide halohydrique en libérant l'halogène, et qu'on récupère l'acide carboxylique formé.

2. Procédé selon la revendication 1, caractérisé en ce que l'aldéhyde de départ répond à la formule RCHO, dans laquelle R représente :

- un groupe hydrocarboné de 1 à 30 atomes de carbone, linéaire ou ramifié, comportant le cas échéant dans la chaîne, un ou plusieurs atomes d'oxygène et/ou un ou plusieurs groupes oxo, choisi parmi les groupes alcoyle, alcoxy ou acyle,
- un groupe cycloalcoyle de 1 à 30 atomes de carbone,
- un groupe acyle comprenant un ou plusieurs cycles aromatiques,
- un groupe arylacyle où les parties aryle et acyle sont respectivement telles que définies ci-dessus,
- un groupe hétérocyclique,

ces différents groupes étant le cas échéant substitués par un ou plusieurs groupements inertes dans les conditions réactionnelles, tels que des atomes d'halogène, des groupes alcoyle, alcoxy dans lesquels la partie alcoyle linéaire ou ramifiée comporte de 1 à 5 atomes de carbone, - COOA dans lequel A représente un groupe alcoyle de 1 à 5 atomes de carbone, aryloxy, benzo et nitro.

3. Procédé selon la revendication 1 ou 2, caractérisisé en ce que le composé peroxydé est choisi parmi les peroxydes capables d'oxyder les acides halohydriques en halogène correspondant tel que :

- le peroxyde d'hydrogène,
- un peroxyde capable de libérer du peroxyde d'hydrogène en milieu aqueux, tel l'anion peroxyborate,
- les hydroperoxydates et en particulier les dérivés de l'urée ou des sels alcalins et alcalino-terreux des acides par exemple carboniques, siliciques ou pyrophosphoriques,
- les sels de l'acide diperoxysulfurique, les peroxyacides organiques, l'acide monoperoxysulfurique et ses sels (dans les cas où le réarrangement de BAEYER VILLIGER n'est pas la réaction majoritaire),
- les composés à fonction hydroperoxyde tels que par exemple l'hydroperoxyde de tertiobutyle.

4. Procédé selon la revendication 3, caractérisé en ce qu'on utilise $H_2O_2$ selon une concentration de 1 à 85% en poids et de préférence de 30 à 35% environ.

5. Procédé selon la revendication 3 ou 4, caractérisé en ce que le rapport molaire $H_2O_2$/RCHO varie dans le domaine 0,5/1 à 3/1 et de préférence est de 2/1.

6. Procédé selon l'une quelconque des revendications 1 à 5, caractérisé en ce que l'acide halohydrique HX est choisi parmi HCl ou HBr.

7. Procédé selon la revendication 6, caractérisé en ce que l'acide halohydrique est utilisé sous forme concentrée, de préférence à raison d'au moins environ 47% en poids pour HBr et 37% en poids pour HCl.

8. Procédé selon l'une quelconque des revendications 1 à 7, caractérisé en ce que le rapport molaire HX:RCHO est au minimum de 0,005:1 et de préférence 0,09:1 à 0,39:1.

9. Procédé selon l'une quelconque des revendications 1 à 8, caractérisé en ce que le pH est acide et diminué jusqu'à une valeur inférieure ou égale à 5 lorsque le composé peroxydé est un peroxyde minéral ou un hydroperoxydate à réaction alcaline en solution aqueuse.

10. Procédé selon l'une quelconque des revendications 1 à 9, caractérisé en ce qu'on utilise un solvant organique à fonction éther tel que le dioxanne 1-4, ou un alcool aliphatique tertiaire.